# EUROPÄISCHE PATENTSCHRIFT

(11) **EP 0 771 345 B1**
(45) Veröffentlichungstag und Bekanntmachung des Hinweises auf die Patenterteilung: **10.01.2001**
(21) Anmeldenummer: 95943545.4
(22) Anmeldetag: 07.07.1995
(51) Int. Cl.: C11C 3/12, C07C 27/02, C07C 29/149, C07C 33/025, C11D 1/66

(54) **UNGESÄTTIGTE FETTSTOFFE MIT VERBESSERTEM KÄLTEVERHALTEN**
UNSATURATED FATS WITH IMPROVED LOW-TEMPERATURE BEHAVIOUR
PRODUITS GRAS INSATURES A COMPORTEMENT AMELIORE AU FROID

(30) Priorität: 16.07.1994 DE 4425180
(43) Veröffentlichungstag der Anmeldung: 07.05.1997
(73) Patentinhaber: Cognis Deutschland GmbH, 40589 Düsseldorf (DE)
(72) Erfinder: KÖHLER, Michael, D-40822 Mettmann (DE); WESTFECHTEL, Alfred, D-40723 Hilden (DE); DEMMERING, Günther, D-42653 Solingen (DE); KOMP, Horst-Dieter, D-40764 Langenfeld (DE); BÖHR, Christiane, D-51379 Leverkusen (DE); ANSMANN, Achim, D-40699 Erkrath (DE); STEINBERNER, Udo, D-40724 Hilden (DE)
(86) Internationale Anmeldenummer: EP9502646
(87) Internationale Veröffentlichungsnummer: WO9602619

(56) Entgegenhaltungen:
- EP-A- 0 370 273
- DE-A- 4 129 622
- DE-B- 1 228 603
- US-A- 4 338 221

## Beschreibung

Die Erfindung betrifft ungesättigte Fettstoffe mit verbessertem Kälteverhalten, die man erhält, indem man ausgewählte Pflanzenöle in Fettsäuren und Glycerin spaltet, die resultierenden Spaltfettsäuren einer fraktionierten Kristallisation unterwirft und die dabei anfallende Fraktion weitgehend ungesättigter Fettsäuren, gegebenenfalls nach Überführung in die Methylester, unter Erhalt der Doppelbindugen zu den entsprechenden Fettalkoholen hydriert. Die Erfindung erstreckt sich ferner auf Derivate der ungesättigten Fettalkohole sowie auf Verfahren zu deren Herstellung und deren Verwendung zur Herstellung oberflächenaktiver Mittel. Ein letzter Gegenstand der Erfindung betrifft schließlich die Verwendung ausgewählter Pflanzenöle zur Herstellung ungesättigter Fettalkohole.

### Stand der Technik

Fettstoffe, insbesondere ungesättigte Fettalkohole, stellen wichtige Zwischenprodukte für eine große Anzahl von Erzeugnissen der chemischen Industrie dar, wie z.B. für die Herstellung von Tensiden und kosmetischen Produkten. Eine Übersicht zu diesem Thema findet sich beispielsweise von U.Ploog et al. in **Seifen-Öle-Fette-Wachse 109, 225 (1983).**

Die Herstellung ungesättigter Fettalkohole gelingt nicht auf Basis petrochemischer Rohstoffe und Verfahren. Man geht vielmehr von mehr oder minder ungesättigten Fettsäuren oder deren Methylestern auf Basis nachwachsender Rohstoffe aus, die beispielsweise in Gegenwart von chrom- und/oder zinkhaltigen Mischoxidkatalysatoren unter Erhalt der Doppelbindungen hydriert werden [vgl. **Ullmam's Enzyklopaedie der technischen Chemie, Verlag Chemie, Weinheim, 4. Aufl., Bd.11, S. 436f].**

Die Herstellung ungesättigter Fettalkohole kann grundsätzlich auf drei Wegen erfolgen:
1. Fette und Öle werden einer Druckspaltung mit Wasser unterworfen. Nach Abtrennung des wäßrigen Glycerins werden Spaltfettsäuren erhalten, die Gemische gesättigter und ungesättigter Fettsäuren darstellen. Da eine gemeinsame Hydrierung dieser Säuren das Verhältnis gesättigter und ungesättigter Anteile nicht beeinflußen kann, werden auf diesem Wege nur Fettalkohole einer niedrigen Iodzahl im Bereich kleiner 85 erhalten, die weniger bevorzugt sind.
2. Eine destillative Trennung gesättigter und ungesättigter C_{16/18}-Fettsäuren ist destillativ nicht möglich. Abweichend von (1) können die Spaltfettsäuren jedoch über den Weg der "Umnetztren-ung" in einen überwiegend gesättigten und einen überwiegend ungesättigten Fettsäureschnitt überführt werden. Die Hydrierung des ungesättigten Fettsäureanteils liefert technische Oleylalkohole eines Iodzahlbereiches von etwa 85 bis 100.
3. Weiterhin ist es möglich, hochungesättigte Pflanzenöle einer Umesterung zu unterwerfen, bei der Methylester mit einem vergleichsweise geringen Anteil an gesättigten Homologen anfallen. Eine Umnetztrennung ist in diesem Fall weder möglich, noch erforderlich, da die Hydrierung unmittelbar hochungesättigte Fettalkohole (IZ > 100) liefert.

Die drei genannten Verfahren werden seit langem kommerziell zur Herstellung von ungesättigten Fettalkoholen genutzt. Es ist naheliegend, zur Herstellung von ungesättigten Fettalkoholen auch Ausgangsstoffe einzusetzen, die bereits eine hohe Iodzahl aufweisen.

Beispiele für geeignete Einsatzprodukte gemäß Verfahren 1 sind Fette und Öle mit einer Iodzahl im Bereich von 40 bis 70, wie beispielsweise Rindertalg, Schweineschmalz, Palmöl oder Palmstearin. Für die Herstellung hochungesättigter Fettalkohole nach Verfahren 3 kommen z.B. Rapsöl, Olivenöl, Sonnenblumenöl, Leinöl oder Erdnußöl in Frage.

Einsatzstoffe wie beispielsweise Kokos- oder Palmkernöl sind bislang zur Herstellung von ungesättigten Fettalkoholen des Iodzahlbereiches 90 bis 100 nicht in Betracht gezogen worden, da sie einen zu geringen Anteil ungesättigter Species enthalten.

Die nach den oben geschilderten Verfahren erhältlichen ungesättigten Fettalkohole des Marktes weisen jedoch verschiedene Nachteile auf: Produkte mit einer Iodzahl unterhalb von 80 sind wachsartig. Neben des unvorteilhaften Erstarrungspunktes weisen sie die Vorteile, die mit der ungesättigten Struktur verbunden sind, naturgemäß nur partiell auf. Hochungesättigte Fettalkohole (Iodzahl > 100) enthalten einen signifikanten Anteil an mehrfach ungesättigten Homologen und sind damit weniger autoxidationsstabil. Die Produkte sind zwar flüssig, lassen sich jedoch nur mit Schwierigkeiten in cremige oder pastöse Formulierungen einarbeiten. Aus anwendungstechnischer Sicht kommen daher am ehesten ungesättigte Fettalkohole des Iodzahlbereiches 85 bis 100 in Frage. Diese sind jedoch oftmals sowohl bezüglich ihrer Farbe als auch der Geruchsqualität nicht zufriedenstellend und weisen für viele Anwendungen einen ebenfalls unvorteilhaft hohen Erstarrungspunkt auf.

Die Aufgabe der Erfindung hat somit darin bestanden, ungesättigte Fettalkohole mit einer Iodzahl im Bereich von 85 bis 100 auf Basis pflanzlicher Rohstoffe - sowie entsprechende Folgeprodukte - zur Verfügung zu stellen, die sich insbesondere durch ein verbessertes Kälteverhalten sowie Farb- und Geruchsqualität auszeichnen.

### Beschreibung der Erfindung

Gegenstand der Erfindung sind ungesättigte Fettstoffe mit verbessertem Kälteverhalten, dadurch erhältlich, daß man
(a) Lauricöle in Fettsäuren und Glycerin spaltet,
(b) die resultierenden Spaltfettsäuren einer fraktionierten Kristallisation unterwirft und
(c) die Fraktion der überwiegend ungesättigten Fettsäuren, gegebenenfalls nach Überführung in die Niedrigalkylester zu den entsprechenden ungesättigten Fettalkoholen im Iodzahlbereich 85 bis 100 und vorzugsweise 90 bis 95 hydriert.

Überraschenderweise wurde gefunden, daß durch den Einsatz von ausgewählten Pflanzenölen eines niedrigen Iodzahlbereiches sowie die Aufreinigung der auf dieser Basis als Zwischenstufen hergestellten ungesättigten Fettsäuren in der Hydrierung erstmals ungesättigte, pflanzliche Fettalkohole erhalten werden, die nicht nur außergewöhnlich gute Farb- und Geruchseigenschaften aufweisen, sondern sich zudem noch in gewünschter Weise durch ein besonders vorteilhaftes Kälteverhalten auszeichnen. Der Einsatz der genannten niederiodzahligen Rohstoffe in ein an sich bekanntes Verfahren, das aus ökonomischen Gründen bislang nur für die Anwendung auf Rohstoffe mit deutlichen höheren Iodzahlen bekannt gewesen ist, erscheint im Hinblick auf die gefundenen überraschenden Effekte neu und erfinderisch.

Weitere vorteilhafte Ausgestaltungen der Erfindung bestehen ferner in Derivaten mit ebenfalls vorteilhaftem Kälteverhalten sowie verbesserten Farb- und Geruchseigenschaften, die man erhält, indem man die eingangs genannten ungesättigten Fettalkohole in an sich bekannter Weise
*** alkoxyliert;
*** alkoxyliert, sulfatiert und neutralisiert;
*** sulfatiert und neutralisiert; bzw.
*** mit aliphatischen Carbonsäuren mit 1 bis 22 Kohlenstoffatomen und 0 und/oder 1 bis 3 Doppelbindungen verestert.

### Herstellverfahren

Ein weiterer Gegenstand der Erfindung betrifft ein Verfahren zur Herstellung ungesättigter Fettstoffe mit verbessertem Kälteverhalten, bei dem man
(a) Lauricöle in Fettsäuren und Glycerin spaltet,
(b) die resultierenden Spaltfettsäuren einer fraktionierten Kristallisation unterwirft und
(c) die Fraktion der überwiegend ungesättigten Fettsäuren, gegebenenfalls nach Überführung in die Niedrigalkylester zu den entsprechenden ungesättigten Fettalkoholen im Iodzahlbereich 85 bis 100 und vorzugsweise 90 bis 95 hydriert.

### Lauricöle

Unter Lauricölen sind Palmkern-, Kokos- und Babassuöl sowie deren Abmischungen zu verstehen, die einen Schwerpunkt ihrer C-Kettenverteilung im Bereich C₁₂ bis C₁₄ aufweisen. Typische Zusammensetzungen sind in Tabelle 1 gegeben:

**Tabelle 1**

| Typische Zusammensetzung Palmkern- und Kokosöl | | |
|---|---|---|
| **Fettsäurekomponente** | **Palmkernöl Gew.-%** | **Kokosöl Gew.-%** |
| Capronsäure | 0 - 1 | 0 - 1 |
| Caprylsäure | 3 - 10 | 6 - 9 |
| Caprinsäure | 3 - 14 | 6 - 10 |
| Laurinsäure | 37 - 52 | 44 - 51 |
| Myristinsäure | 7 - 17 | 13 - 18 |
| Palmitinsäure | 2 - 9 | 8 - 10 |
| Stearinsäure | 1 - 3 | 1 - 3 |
| Ölsäure | 11 - 23 | 6 - 8 |
| Linolsäure | 1 - 3 | 0 - 3 |
| Verseifungszahl | 245 - 255 | 250 - 264 |
| Iodzahl | 14 - 23 | 8 - 11 |
| Schmelzpunkt (°C) | 26 - 26 | 23 - 26 |

Im Sinne der Erfindung sind andere Fette und öle bzw. deren Abmischungen, die ebenfalls Iodzahlen im Bereich von 5 bis 25, einen Gehalt an C₁₂-C₁₄-Fettsäuren im Bereich von 30 bis 55 Gew.-% und einen Linol(en)säuregehalt von in Summe < 5 Gew.-% aufweisen, als glatte Äquivalente anzusehen, die von der vorliegenden Lehre mitumfaßt werden.

Bevorzugt ist der Einsatz von Palmkernöl, auf dessen Basis sich im Sinne des erfindungsgemäßen Verfahrens ein technischer Oleylalkohol mit einer Iodzahl von 94, einem Erstarrungspunkt von 7°C, einer Farbe von 10 Hazen und einer Zusammensetzung von 5 Gew.-% C₁₆ und 95 Gew.-% C₁₈ herstellen läßt.

### Spaltung

Unter dem Begriff Spaltung ist die Verseifung bzw. Hydrolyse von Glycerinfettsäure(partial)estern zu Glycerin und Fettsäuren zu verstehen. Das Verfahren geht auf E.Twitchell zurück, der 1898 ein Normaldruckverfahren zur Spaltung von Triglyceriden entwickelt hat, bei der die Hydrolyse in Gegenwart von Schwefelsäure und einem Naphthalin/Ölsäure-Gemisch (TwitchellReagenz) durchgeführt wurde. Seit der Entwicklung von geeigneten Edelstählen in den 30er Jahren wird die seit 1854 bekannte Druckspaltung von Fetten auch großtechnisch mit Wasserdampf durchgeführt [vgl. **Fat Sci. Technol., 89, 297 (1987).** Letztere ist neben dem EMERSOL-Verfahren im Sinne der Erfindung bevorzugt.

### Fraktionierte Kristallisation und Umnetztrennuna

Die Trennung von gesättigten und ungesättigten C_{16/18}-Fettsäuren wird als fraktionierte Kristallisation durchgeführt. Vorzugsweise handelt es sich hierbei um eine Umnetztrennung.

Unter dem Begriff Umnetztrennung ("Rolling-up Process") versteht man die Trennung von gesättigten und ungesättigten Fettsäuren mit in der Regel 12 bis 18 und vorzugsweise 16 bis 18 Kohlenstoffatomen, die auf destillativem Wege infolge der sehr ähnlichen Siedepunkte nicht oder nur mit hohem technischen Aufwand durchgeführt werden kann. Eine Übersicht zu diesem Thema ist von K.Schmid in **Fat Sci.Technol. 89, 237 (1987)** veröffentlicht worden.

Das Verfahren der Umnetztrennung wurde zu Beginn der 60er Jahre für die Trennung von Talgfettsäure in einen C₁₆/₁₈-Palmitin/Stearinschnitt ("Stearin") und einen ölsäureanteil ("Olein") entwickelt, ist jedoch auch auf Spaltfettsäuren einer breiteren C-Kettenverteilung anwendbar. Für der Fall der Talgfettsäure arbeitet das Verfahren in der Weise, daß die technische Fettsäuremischung zunächst auf niedrigen Temperaturen von ca. 5°C abgekühlt wird, wobei eine Kristallisation der Palmitin-/Stearinsäure in der flüssigen Ölsäure unter Bildung einer Dispersion stattfindet. An dieser Stelle könnte zwar bereits eine physikalische Trennung durchgeführt werden, es zeigt sich jedoch, daß beispielsweise bei einer Filtration zu große Anteile von Ölsäure an den Palmitin-/ Stearinsäurekristallen anhaften. Um die Ölsäure von den Kristallen "abzuwaschen" setzt man der Dispersion eine wäßrige Netzmittellösung - beispielsweise ein wäßriges Alkylsulfat - zu. Beim anschließenden Zentrifugieren dieser Emulsion/Dispersion erfolgt eine Spaltung der Emulsion in eine Ölsäurephase und eine Wasser/gesättigte Fettsäuren-Dispersion, die in einem Separator getrennt werden können. Die Palmitin-/ Stearinsäure-Wasserdispersion wird dann auf etwa 50 bis 80°C erwärmt und die geschmolzene Palmitin-/Stearinsäure von der wäßrigen Netzmittellösung abgetrennt, die in den Prozeß zurückgeführt wird.

### Hydrierung

Die Reduktion von Estern mit metallischem Natrium in Gegenart eines Alkohols wurde schon 1903 von Bouveault und Blanc entdeckt. Die großtechnische Herstellung von Fettalkoholen erfolgt heute jedoch praktisch ausschließlich durch Hochdruckhydrierung von destillierten bzw. fraktionierten Methylester-bzw. Fettsäureschnitten in einem oder mehreren hintereinandergeschalteten Festbett- oder Schachtreaktoren bei Temperaturen von 200 bis 250°C und einem Wasserstoffdruck von 200 bis 300 bar. Dazu werden Fettsäure oder C₁-C₄-Niedrigalkylester, vorzugsweise jedoch Methylester, kontinuierlich gegen den Wasserstoffdruck in die Anlage gepreßt, auf Reaktionstemperatur erhitzt und am Reaktorkopf aufgegeben. Zur Herstellung ungesättigter Fettalkohole werden üblicherweise Adkins-Katalysatorschüttungen auf Basis von Cu/Cr/Zn und/oder Cu/ Cr/Cd-Mischoxiden eingesetzt; in diesem Fall kommt es zu einer selektiven Hydrierung der Carboxyl(at)gruppe unter Erhalt der im Fettrest enthaltenen Doppelbindungen.

Neben der Festbettfahrweise kann die Hydrierung auch in der Rieselphase durchgeführt werden. Auch bei dieser Verfahrensvariante durchströmen Fettsäure bzw. Ester und Wasserstoff den Reaktor bei einer Temperatur von 200 bis 300°C und einem Druck von 250 bis 300 bar von oben. Jedoch sind hier die Kreisgasmengen und der molare Wasserstoffüberschuß erheblich geringer, was sich in kleineren Anlagendimensionen wiederspiegelt. Als Katalysatoren werden Silikagel-Trägerkontakte mit 20 bis 40 Gew.-% der eingangs genannten Cupferchromite verwendet. Diese Katalysatoren besitzen zwar eine hohe mechanische Stabilität, sind jedoch wegen ihres geringen Gehaltes an Aktivsubstanz anfälliger gegen Vergiftungen als Massivkontakte und besitzen daher kürzere Standzeiten.

Vorzugsweise werden die resultierenden Fettalkohole anschließend unter Abnahme eines Vorlaufes (etwa 5 Gew.-%) in an sich bekannter Weise destillativ gereingt.

Falls gewünscht, kann sich an die Reinigung eine erneute fraktionierte Kristallisation ("Winterisierung") anschließen.

### Abmischungen

In einer weiteren vorteilhaften Ausführungsform der Erfindung können die neuen ungesättigten Fettalkohole auf Basis von Palmkern- und/oder Kokosöl konventionellen gesättigten und/ oder ungesättigten Fettalkoholen mit 6 bis 22 und vorzugsweise 16 bis 18 Kohlenstoffatomen zugemischt werden. Der Vorteil besteht darin, daß die Mischungen ebenfalls verbesserte anwendungstechnische Eigenschaften aufweisen.

So weist beispielsweise eine Mischung aus 60 bis 65 Gew.-% eines erfindungsgemäßen technischen Oleylalkohols auf Basis Palmkernöl und 35 bis 40 Gew.-% eines konventionellen Oleylalkohols (HD Ocenol^{(R)} 60/65, Henkel KGaA) einen Erstarrungspunkt unterhalb von 20°C auf; der Erstarrungspunkt eines von seiner Zusammensetzung her vergleichbaren Oleylalkohols (HD Ocenol^{(R)} 70/75, Henkel KGaA) auf Basis Rindertalg liegt hingegen bei 22°C.

### Derivatisierung

Wie schon eingangs geschildert, besteht eine weitere Erkenntnis der vorliegenden Erfindung darin, daß die hervorragenden Eigenschaften der primär hergestellten ungesättigten Fettalkohole auch nach Derivatisierung erhalten bleiben. Hierzu zählen:
*** **Alkoxylierung.** Alkoxylate der ungesättigten Fettalkohole werden an sich bekannter Weise durch Anlagerung von Ethylen- und/oder Propylenoxid in Gegenwart basischer Katalysatoren wie z.B. Natriummethylat oder calcinierter Hydrotalcit erhalten und können sowohl eine konventionelle als auch eine eingeengte Homologenverteilung aufweisen. Die Alkoxylate eignen sich z.B. als Waschmittelrohstoffe, Emulgatoren im Bereich der Textiltechnik, bei Bohr- und Schneidölen sowie in kosmetischen Formulierungen.
*** **Alkoxylierung/Sulfatierung.** Ethersulfate der ungesättigten Fettalkohole werden in an sich bekannter Weise durch Alkoxylierung, nachfolgende Sulfatierung mit gasförmigem Schwefeltrioxid oder Chlorsulfonsäure sowie abschließende Neutralisation mit Basen erhalten. Die Produkte eignen sich als Waschrohstoffe.
*** **Sulfatierung.** Fettalkoholsulfate auf Basis der ungesättigten Alkohole werden in an sich bekannter Weise durch Sulfatierung mit gasförmigem Schwefeltrioxid oder Chlorsulfonsäure sowie abschließende Neutralisation mit Basen erhalten. Die Produkte eignen sich ebenfalls als Waschrohstoffe und Hilfsmittel in der Textiltechnik.
*** **Veresterung.** Ester der ungesättigten Fettalkohole werden in an sich bekannter Weise durch katalytische Umsetzung mit aliphatischen Carbonsäuren mit 1 bis 22, vorzugsweise 6 bis 22 und insbesondere 12 bis 18 Kohlenstoffatomen und 0 und/oder 1 bis 3 Doppelbindungen erhalten. Typische Beispiele sind Umsetzungen eines erfindungsgemäßen technischen Oleylalkohols (Iodzahl 95) mit Essigsäure, C₆-C₁₀-Vorlauffettsäure, Laurinsäure, Palmitinsäure, Stearinsäure, Ölsäure, C_{12/14}-Kokosfettsäure, C_{12/18}-Kokosfettsäure oder C_{16/18}-Talgfettsäure. Vorzugsweise wird die Veresterung mit Ölsäure unter Bildung eines Oleyloleats durchgeführt. Die Produkte eignen sich beispielsweise als Ölkörper zur Herstellung kosmetischer Mittel.

Weitere Derivatisierungen, die ebenfalls in Betracht kommen, sind Amidierung, Phosphatierung, die Bildung von Addukten mit Maleinsäureanhydrid sowie die Epoxidation, vorzugsweise nach Überführung der Oleylalkohole in die Methylester.

### Gewerbliche Anwendbarkeit

Die erfindungsgemäßen ungesättigten Fettstoffe zeichnen sich gegenüber den Produkten des Stands der Technik durch einen verbesserten Geruch, eine verbesserte Farbe und insbesondere durch ein vorteilhafteres Kälteverhalten aus.

Ein weiterer Gegenstand der Erfindung betrifft ihre Verwendung zur Herstellung oberflächenaktiver Mittel, wie z.B. Überfettungs- oder Lösungsmittel für Wirkstoffe, Cremes, Salben und Lotionen, Schmiermittel in der Metallbearbeitung sowie Antischaummittel in Dispersionsfarben, in denen sie in Mengen von 1 bis 75, vorzugsweise 5 bis 50 Gew.-% - bezogen auf die Mittel - enthalten sein können.

Ein weiterer Gegenstand der Erfindung betrifft schließlich die Verwendung von Lauricölen zur Herstellung von ungesättigten Fettalkoholen im Iodzahlbereich von 85 bis 100 und vorzugsweise 90 bis 95 über den Weg der Spaltung, fraktionierten Kristallisation und Hydrierung.

### Beispiele

### Allgemeine Herstellvorschriften

**Verfahren 1.** Die Einsatzstoffe wurden einer Druckspaltung unterworfen und das Glycerin/Wasser-Gemisch abgetrennt. Das resultierene Spaltfettsäurengemisch wurde nach Überführung in die Methylester ohne weitere Aufreinigung unter Erhalt der Doppelbindungen hydriert. Anschließend wurde das Produkt durch Destillation gereinigt.

**Verfahren 2.** Die Einsatzstoffe wurden einer Druckspaltung unterworfen und das Glycerin/Wasser-Gemisch abgetrennt. Das resultierene Spaltfettsäurengemisch wurde in der Umnetztrennung in einen Stearin- und einen Oleinanteil aufgetrennt. Das Olein, das etwa 80 Gew.-% Ölsäure enthielt, wurde mit Methanol verestert und im Anschluß unter Erhalt der Doppelbindungen hydriert. Anschließend wurde das Produkt durch Destillation gereinigt.

**Verfahren 3.** Die Einsatzstoffe wurden einer Umesterung mit Methanol unterworfen und von Glycerin und nicht umgesetztem Alkohol befreit. Das resultierende Methylestergemisch wurde ohne weitere Aufreinigung unter Erhalt der Doppelbindungen hydriert. Anschließend wurde das Produkt durch Destillation gereinigt.

Die Zusammensetzung der eingesetzten Rohstoffe ist in Tabelle 2 wiedergegeben (Prozentangaben als Gew.-%). Tabelle 3 können die anwendungstechnischen Daten der resultierenden ungesättigten Fettalkohole entnommen werden.

**Tabelle 2**

| Eingesetzte Rohstoffe | | | | | | |
|---|---|---|---|---|---|---|
| **Fettsäurekomponente** | **PK** % | **KK %** | **RT %** | **PM %** | **PS %** | **OL %** |
| Capronsäure | 1 | 1 | 0 | 0 | 0 | 0 |
| Caprylsäure | 4 | 8 | 0 | 0 | 0 | 0 |
| Caprinsäure | 5 | 7 | 0 | 0 | 0 | 0 |
| Laurinsäure | 50 | 48 | 0 | 0 | 0 | 0 |
| Myristinsäure | 15 | 17 | 3 | 2 | 4 | 10 |
| Palmitinsäure | 7 | 9 | 27 | 42 | 72 | 3 |
| Stearinsäure | 2 | 2 | 22 | 5 | 10 | 0 |
| Ölsäure | 15 | 7 | 43 | 41 | 11 | 80 |
| Linolsäure | 1 | 1 | 5 | 10 | 3 | 7 |
| Iodzahl | 19 | 9 | 44 | 50 | 15 | 82 |
| Legende: PK = Palmkernöl PM = Palmöl KK = Kokosöl PS = Palmstearin RT = Rindertalg OL = Olivenöl | | | | | | |

**Tabelle 3**

| Kenndaten der Produkte | | | | | | |
|---|---|---|---|---|---|---|
| **Bsp.** | **Rohstoff** | **Ungesättigter Fettalkohol** | | | | |
| | | **IZ** | **Verf.** | **EP °C** | **Farbe Hazen** | **Geruch** |
| 1 | Palmkernöl | 94 | 2 | 7,0 | 10 | +++ |
| 2 | Kokosöl | 91 | 2 | 8,5 | 15 | ++ |
| V1 | Palmkernöl | 35 | 1 | 30,5 | 20 | + |
| V2 | Kokosöl | 35 | 1 | 32,0 | 25 | + |
| V3 | Rindertalg | 94 | 2 | 14,5 | 85 | - |
| V4 | Palmöl | 94 | 2 | 15,5 | 90 | - |
| V5 | Palmstearin | 94 | 2 | 17,0 | 90 | - |
| V6 | Olivenöl | 112 | 2 | 7,0 | 25 | + |
| V7 | Olivenöl | 108 | 3 | 7,0 | 120 | + |
| Legende: IZ = Iodzahl Verf. = Verfahrenstyp EP = Erstarrungspunkt Geruch = +++ = geruchsfrei ++ = Geruch kaum wahrnehmbar + = Geruch wahrnehmbar - = Geruch deutlich wahrnehmbar | | | | | | |

### Beispiel 3:

Der ungesättigte Oleylalkohol aus Beispiel 1 wurde mit Palmkernfettsäure (Edenor^{(R)} PK 1805, Henkel KGaA) verestert. Der resultierende Ester wies folgende Kennzahlen auf:

| | |
|---|---|
| Hydroxylzahl | 209 |
| Iodzahl | 94 |
| Verseifungszahl | 0,3 |
| Erstarrungspunkt | 7,2 |
| Farbzahl (APHA) | 10 |

## Patentansprüche

1. Ungesättigte Fettstoffe mit verbessertem Kälteverhalten, dadurch erhältlich, daß man
(a) Lauricöle in Fettsäuren und Glycerin spaltet,
(b) die resultierenden Spaltfettsäuren einer fraktionierten Kristallisation unterwirft und
(c) die Fraktion der überwiegend ungesättigten Fettsäuren, gegebenenfalls nach Überführung in die Niedrigalkylester zu den entsprechenden ungesättigten Fettalkoholen im Iodzahlbereich 85 bis 100 hydriert.

2. Fettstoffe nach Anspruch 1, **dadurch gekennzeichnet,** daß man die ungesättigten Fettalkohole anschließend in an sich bekannter Weise alkoxyliert.

3. Fettstoffe nach Anspruch 1, **dadurch gekennzeichnet,** daß man die ungesättigten Fettalkohole anschließend in an sich bekannter Weise alkoxyliert, sulfatiert und neutralisiert.

4. Fettstoffe nach Anspruch 1, **dadurch gekennzeichnet,** daß man die ungesättigten Fettalkohole anschließend in an sich bekannter Weise sulfatiert und neutralisiert.

5. Fettstoffe nach Anspruch 1, **dadurch gekennzeichnet,** daß man die ungesättigten Fettalkohole anschließend in an sich bekannter Weise mit aliphatischen Carbonsäuren mit 1 bis 22 Kohlenstoffatomen und 0 und/oder 1 bis 3 Doppelbindungen verestert.

6. Verfahren zur Herstellung ungesättigter Fettstoffe mit verbessertem Kälteverhalten, **dadurch gekennzeichnet,** daß man
(a) Lauricöle in Fettsäuren und Glycerin spaltet,
(b) die resultierenden Spaltfettsäuren einer fraktionierten Kristallisation unterwirft und
(c) die Fraktion der überwiegend ungesättigten Fettsäuren, gegebenenfalls nach Überführung in die Niedrigalkylester zu den entsprechenden ungesättigten Fettalkoholen im Iodzahlbereich 85 bis 100 hydriert.

7. Verfahren nach Anspruch 6, **dadurch gekennzeichnet,** daß man als Lauricöle Palmkernöl und/oder Kokosöl einsetzt.

8. Verfahren nach den Ansprüchen 6 und 7, **dadurch gekennzeichnet,** daß man die Lauricöle einer Druckspaltung mit Wasser unterwirft.

9. Verfahren nach den Ansprüchen 6 bis 8, **dadurch gekennzeichnet,** daß man die fraktionierte Kristallisation nach dem Umnetzverfahren durchführt.

10. Verfahren nach Anspruch 6, **dadurch gekennzeichnet,** daß man die ungesättigten Fettalkohole anschließend in an sich bekannter Weise alkoxyliert.

11. Verfahren nach Anspruch 6, **dadurch gekennzeichnet,** daß man die ungesättigten Fettalkohole anschließend in an sich bekannter Weise alkoxyliert, sulfatiert und neutralisiert.

12. Verfahren nach Anspruch 6, **dadurch gekennzeichnet,** daß man die ungesättigten Fettalkohole anschließend in an sich bekannter Weise sulfatiert und neutralisiert.

13. Verfahren nach Anspruch 6, **dadurch gekennzeichnet,** daß man die ungesättigten Fettalkohole anschließend in an sich bekannter Weise mit aliphatischen Carbonsäuren mit 1 bis 22 Kohlenstoffatomen und 0 und/oder 1 bis 3 Doppelbindungen verestert.

14. Verfahren nach den Anspruch 6, **dadurch gekennzeichnet,** daß man die ungesättigten Fettalkohole mit weiteren gesättigten und/oder ungesättigten Fettalkoholen mit 6 bis 22 Kohlenstoffatomen abmischt.

15. Verwendung von ungesättigten Fettstoffen nach den Ansprüchen 1 bis 5 zur Herstellung oberflächenaktiver Mittel.

16. Verwendung von Lauricölen zur Herstellung ungesättigter Fettalkohole im Iodzahlbereich 85 bis 100 über den Weg der Spaltung, fraktionierten Kristallisation und Hydrierung nach Anspruch 1.

## Claims

1. Unsaturated fatty compounds with improved low-temperature behavior obtainable by
(a) splitting lauric oils into fatty acids and glycerol,
(b) subjecting the resulting split fatty acids to fractional crystallization and
(c) hydrogenating the fraction of predominantly unsaturated fatty acids, optionally after conversion into the lower alkyl esters, to form the corresponding unsaturated fatty alcohols with iodine values in the range from 85 to 100.

2. Fatty compounds as claimed in claim 1, characterized in that the unsaturated fatty alcohols are subsequently alkoxylated in known manner.

3. Fatty compounds as claimed in claim 1, characterized in that the unsaturated fatty alcohols are subsequently alkoxylated, sulfated and neutralized in known manner.

4. Fatty compounds as claimed in claim 1, characterized in that the unsaturated fatty alcohols are subsequently sulfated and neutralized in known manner.

5. Fatty compounds as claimed in claim 1, characterized in that the unsaturated fatty alcohols are subsequently esterified in known manner with aliphatic carboxylic acids containing 1 to 22 carbon atoms and 0 and/or 1 to 3 double bonds.

6. A process for the production of unsaturated fatty compounds with improved low-temperature behavior, characterized in that
(a) lauric oils are split into fatty acids and glycerol,
(b) the resulting split fatty acids are subjected to fractional crystallization and
(c) the fraction of predominantly unsaturated fatty acids is hydrogenated, optionally after conversion into the lower alkyl esters, to form the corresponding unsaturated fatty alcohols with iodine values in the range from 85 to 100.

7. A process as claimed in claim 6, characterized in that palm kernel oil and/or coconut oil is/are used as the lauric oils.

8. A process as claimed in claim 6 and 7, characterized in that the lauric oils are subjected to pressure hydrolysis with water.

9. A process as claimed in claims 6 to 8, characterized in that the fractional crystallization is carried out by rolling-up.

10. A process as claimed in claim 6, characterized in that the unsaturated fatty alcohols are subsequently alkoxylated in known manner.

11. A process as claimed in claim 6, characterized in that the unsaturated fatty alcohols are subsequently alkoxylated, sulfated and neutralized in known manner.

12. A process as claimed in claim 6, characterized in that the unsaturated fatty alcohols are subsequently sulfated and neutralized in known manner.

13. A process as claimed in claim 6, characterized in that the unsaturated fatty alcohols are subsequently esterified in known manner with aliphatic carboxylic acids containing 1 to 22 carbon atoms and 0 and/or 1 to 3 double bonds.

14. A process as claimed in claim 6, characterized in that the unsaturated fatty alcohols are mixed with other saturated and/or unsaturated fatty alcohols containing 6 to 22 carbon atoms.

15. The use of the unsaturated fatty compounds claimed in claims 1 to 5 for the production of surface-active formulations.

16. The use of lauric oils for the production of unsaturated fatty alcohols with iodine values of 85 to 100 by splitting, fractional crystallization and hydrogenation in accordance with claim 1.

## Revendications

1. Matières grasses à comportement au froid amélioré, caractérisées en ce que l'on
(a) scinde des huiles lauriques en acides gras et en glycérine, on
(b) soumet les acides gras de scission résultants à une cristallisation fractionnée et on
(c) hydrogène la fraction des acides gras en majeure partie insaturés, éventuellement après conversion en esters alkyliques inférieurs, en alcools gras insaturés correspondants dont l'indice d'iode est compris dans la plage de 85 à 100.

2. Matières grasses selon la revendication 1, **caractérisées en ce que** l'on alcoxyle ensuite les alcools gras insaturés d'une manière connue en soi.

3. Matières grasses selon la revendication 1, **caractérisées en ce que** l'on alcoxyle, sulfate et neutralise ensuite les alcools gras insaturés d'une manière connue en soi.

4. Matières grasses selon la revendication 1, **caractérisées en ce que** l'on sulfate et neutralise ensuite les alcools gras insaturés d'une manière connue en soi.

5. Matières grasses selon la revendication 1, **caractérisées en ce que** l'on estérifie ensuite les alcools gras insaturés, d'une manière connue en soi, avec des acides carboxyliques aliphatiques comportant 1 à 22 atomes de carbone et 0 et/ou 1 à 3 doubles liaisons.

6. Procédé de production de matières grasses insaturées à comportement au froid amélioré, **caractérisé en ce que** l'on
(a) scinde des huiles lauriques en acides gras et en glycérine, on
(b) soumet les acides gras de scission résultants à une cristallisation fractionnée et on
(c) hydrogène la fraction des acides gras en majeure partie insaturés, éventuellement après conversion en esters alkyliques inférieurs, en alcools gras insaturés correspondants dont l'indice d'iode est compris dans la plage de 85 à 100.

7. Procédé selon la revendication 6 , **caractérisé en ce que** l'on utilise comme huile laurique, de l'huile de palmiste et/ou de coco.

8. Procédé selon les revendications 6 et 7, **caractérisé en ce que** l'on soumet l'huile laurique à une scission sous pression à l'eau.

9. Procédé selon les revendications 6 à 8, **caractérisé en ce que** l'on opère la cristallisation fractionnée selon le procédé mettant en oeuvre un agent mouillant.

10. Procédé selon la revendication 6, **caractérisé en ce que** l'on alcoxyle ensuite les alcools gras insaturés d'une manière connue en soi.

11. Procédé selon la revendication 6, **caractérisé en ce que** l'on alcoxyle, sulfate et neutralise ensuite les alcools gras insaturés d'une manière connue en soi.

12. Procédé selon la revendication 6, **caractérisé en ce que** l'on sulfate et neutralise ensuite les alcools gras insaturés d'une manière connue en soi.

13. Procédé selon la revendication 6, **caractérisé en ce que** l'on estérifie ensuite les alcools gras insaturés, d'une manière connue en soi, avec des acides carboxyliques aliphatiques comportant 1 à 22 atomes de carbone et 0 et/ou 1 à 3 doubles liaisons.

14. Procédé selon la revendication 6, **caractérisé en ce que** l'on mélange les alcools gras insaturés avec d'autres alcools gras saturés et/ou insaturés comportant 6 à 22 atomes de carbone.

15. Utilisation des matières grasses insaturées selon les revendications 1 à 5, pour la production d'agents tensioactifs.

16. Utilisation d'huiles lauriques pour la production d'alcools gras insaturés de la plage d'indice d'iode de 85 à 100, par scission, cristallisation fractionnée et hydrogénation.
